# EUROPEAN PATENT APPLICATION

(11) **EP 1 952 845 A1**
(43) Date of publication of application: **06.08.2008**
(21) Application number: 08000479.9
(22) Date of filing: 11.01.2008
(51) Int. Cl.: A61Q 19/08, A61K 8/37, A61K 8/41, A61K 8/42, A61K 8/46, A61K 8/49

(54) **Use of an astaxathin derivative for cosmetic purposes**

(30) Priority: 26.01.2007 EP 07001780
(71) Applicant: DSM IP Assets B.V., 6411 TE Heerlen (NL)
(72) Inventor: Beumer, Raphael, 79541 Loerrach (DE); Klock, Jochen, 79104 Freiburg (DE); Mendrok-Edinger, Christine, 79618 Rheinfelden-Minseln (DE); Pheiffer, Joachim, 79539 Lörrach (DE); Schick, Ute, 5430 Wettingen (CH); Vollhardt, Jürgen H., 4433 Ramlinsburg (CH)
(74) Representative: Pressner, Dietmar

(57) **Abstract**

The present invention is directed to cosmetic compositions comprising an astaxanthin derivative for cosmetic purposes. It was found that said astaxanthin derivatives are particularly useful for the beautification of mammalian skin, hair and nails, in particular as an antiageing agent, as an anti-wrinkle agent, as antioxidant, as antiinflammatory agent, as anti-hyperpigmentation agent, as anti-photoaging agent and/ or as melanin inhibition agent.

## Description

The present invention is directed to cosmetic compositions comprising an astaxanthin derivative for cosmetic purposes. It was found that said astaxanthin derivatives are particularly useful for the beautification of mammalian skin, hair and nails, in particular as an antiageing agent, as an antiwrinkle agent, as antioxidant, as antiinflammatory agent, as anti-hyperpigmentation agent, as anti-photoaging agent and/ or as melanin inhibition agent.

Human skin consists of several compartments, e.g. epidermis, dermis, follicles and subdermis. Specific cell types and their activities contribute to the specific tasks of each compartment. Cells can be found in various stages of differentiation, particularly in the epidermis and dermis. The more pronounced the degree of differentiation, the more specialized and dedicated a cell is. Casual factors or external factors such as a raw climate, wind, irritation by the sun, rain and snow disturb the normal condition of the skin, and in particular the barrier function of the skin. As a net result there appears roughness, formation of scales (for example on the scalp), an excessive keratinization and similar phenomena. The skin becomes unattractive.

Furthermore, in the course of aging of the skin various signs appear that are especially manifested by a change in the structure and function of the skin. One of these signs is the appearance of fine lines and deep wrinkles, the size and number of which increases with age. The microrelief of the skin becomes less uniform and is of unisotropic nature. In parallel with age the skin becomes more sensitive towards disturbing influences, either intrinsic or extrinsic, which may result in itch, redness or even darkened spots, in particular on hands and the facial area due to pigmentation disorders. These unwanted signs may lead to an undesired age judgment of a person.

Cosmetic preparations are essential for skin care. One aim of skin care in the cosmetic sense is to strengthen or rebuild the skin's natural function as a barrier against environmental influences (e.g. dirt, chemicals, microorganisms) and against the loss of endogenous substances (e.g. water, natural fats, electrolytes). If this function becomes impaired, increased resorption of toxic or allergenic substances or attack by microorganisms may result, leading to toxic or allergic skin reactions.

Another aim of skin care is to compensate for the loss by the skin of lipids and water caused by daily washing. This is particularly important when the natural regeneration ability is inadequate. Furthermore, skin care products should protect against environmental influences, in particular against sun and wind, and delay skin aging.

Another aim of skin care is not even only to prevent or to delay skin aging but also to restore functional losses of the skin due to aging effects. Such effects include thinning of the epidermis and decreased waviness of the basal layer, barrier impairment, sensitive skin, decrease of anti-oxidant potential, increased MMP activity, itchiness, likeliness of chronic inflammation, male baldness, hair graying. In general aging results also in changes in the regulation of biochemical processes, impaired recruitment of cells and slowing down in cell renewal.

Strengthening or thickening of the epidermis can rebuild the skin's barrier ability and is therefore of significant cosmetic value. It also is known that this is a major strategy to fight further extrinsic causes of aging.

Of particular importance for anti-aging cosmetics is to inhibit the senescence of skin cells in order to keep their regular metabolic level on a constant and beneficial level.

Thus, the overall aim of skin care is to provide an attractive and beautiful skin, and there is a need for agents and compositions for the beautification and improvement of mammalian, in particular human, skin, hair and nails.

The use of astaxanthin for cosmetic purposes, e.g. for the improvement of skin wrinkles is known and e.g. disclosed in KR 2005116455. However, astaxanthin is not stable in cosmetic preparations and thus the application of astaxanthin for cosmetic purposes is limited.

The problem to be solved by the present invention is the provision of astaxanthin derivatives which are stable when incorporated into cosmetic compositions while providing beneficial cosmetic effects such as the beautification and improvement of mammalian, in particular human, skin, hair and nails.

This problem is solved on the basis of the unexpected finding that certain astaxanthin derivatives are stable when incorporated into cosmetic compositions and are useful for cosmetic purposes, in particular for beautification and improvement of mammalian skin, hair and nails. The beautification and improvement of mammalian skin encompasses in particular the prevention and/ or treatment of wrinkles as well as the treatment of other conditions observed with skin aging due to environmental or other external influences or due to intrinsic ageing, the thickening of the epidermis, the prevention and/ or treatment of hyperpigmentation disorders, and/ or the treatment of inflammatory diseases of the skin such as acne. Furthermore, the astaxanthin derivatives are useful for cosmetic applications due to their moisturizing properties, anti-oxidant properties or colorant properties.

Accordingly, the present invention provides the use of an astaxanthin derivative represented by general formula (I) 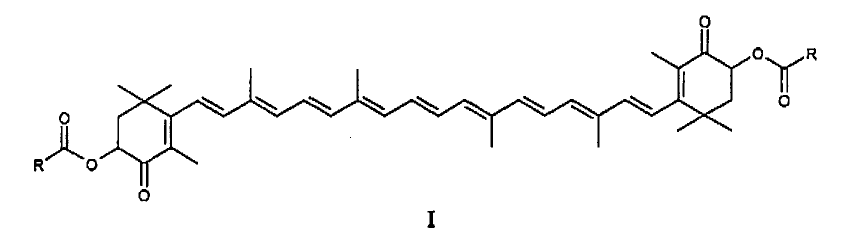 wherein
- R: is in each case a group (a), (b) or (c)

-NH-CH(R¹)-COOR² (a)

-OR³ (b)

-(Y)ₙ-Z (c)
- R¹: signifies hydrogen or the residue of a protein-forming amino acid,
- R²: signifies C₁₋₆-alkyl or C₃₋₈-cycloalkyl,
- R³: signifies C₁₋₁₂-alkyl or C₃₋₈-cycloalkyl,
- n: signifies zero or 1,
- Y: signifies C₁₋₇-alkylene or C₂₋₇-alkenylene,
- and Z: ,when n is zero, signifies C₃₋₈-cycloalkyl, a group -CH(C₆H₅)OR⁴, a group -COR⁵ or a group -CH₂N⁺(CH₃)₃ Hal⁻,
- or Z: ,when n is 1, signifies amino, a group -O-COR⁶, a group -NHCOR⁶, a group -OR⁷ or a group -SR⁸,
- or Z: ,regardless of whether n is zero or 1, signifies alternatively aryl, heteroaryl, a group, -COOR⁵ or a group -CH(CH₃)OR⁴,
- R⁴: signifies hydrogen or acetyl,
- R⁵: signifies hydrogen or C₁₋₆-alkyl.
- R⁶: signifies C₁₋₆-alkyl, aryl or heteroaryl,
- R⁷: signifies hydrogen, C₁₋₆-alkyl or acetyl,
- R⁸: signifies C₁₋₆-alkyl, and
- Hal⁻: signifies a halogen ion
for providing a cosmetic effect, in particular for providing a cosmetic effect by oral or topical application, most preferably by topical application.

In all embodiments of the invention, the astaxanthin derivative is preferably selected from
astaxanthin-diethyidicarbonate (R is ethoxy),
astaxanthin-diethyldioxalate (R is ethoxycarbonyl),
astaxanthin-di(N-acetylglycinate) (R is acetylaminomethyl),
(all-E)-3,3'-rac-astaxanthin-dimaleinate (R is -CH=CH-COOH),
(all-E)-3,3'-rac-astaxanthin-disuccinate (R is -CH₂-CH₂-COOH),
(all-E)-3,3'-astaxanthin-dimethyldisuccinate (R is -CH₂CH₂COOCH₃),
(all-E)-3,3'-rac-astaxanthin-diethyldiglycinedicarbamate (R is -NH-CH₂-COOC₂H₅),
(all-E)-3,3'-rac-astaxanthin-dinicotinate (R is 3-pyridyl), (all-E)-3,3'-rac-astaxanthin-dimethioninedicarbamate (R is -NHCH(CH₂CH₂CH₃)COOC₂H₅),
(all-E)-3,3'-rac-astaxanthin-diacetyidiglycolate (R is acetyloxymethyl),
(all-E)-3.3'-rac-astaxanthin-diphenytalaninedicarbamate (R is -NHCH(CH₂C₆H₅)COOC₂H₅),
(all-E)-3,3'-rac-astaxanthin-diethyldifumarate (R is -CH=CH-COOC₂H₅),
(all-E)-3,3'-rac-astaxanthin-difuran-2-carbonate (R is 2-furyl),
(all-E)-3,3'-rac-astaxanthin-dimethyldimalonate (R is -CH₂-COOCH₃),
(all-E)-3,3'-rac-astaxanthin-di(3-methylthiopropionate) (R is 3-methylthioethyl),
(all-E)-3,3'-rac-astaxanthin-dimethoxyacetate (R is methoxymethyl),
(all-E)-3,3'-rac-astaxanthin-di-[(2-thienyl)acetate] [R is (2-thienyl)methyl],
astaxanthin-dilactate (R is 1-hydroxyethyl),
astaxanthin-di(acetylmandelate) (R is α-acetyloxybenzyl),
(all-E)-3,3'-rac-astaxanthin-dihippurate (R is benzoylaminomethyl) and
(all-E)-3,3'-rac-astaxanthin dibetainate [R is -CH₂N⁺(CH₃)₃Cl⁻].
Most preferably, the astaxanthin derivative is (all-E)-3,3'-astaxanthin-dimethyldisuccinate (R is -CH₂-CH₂-COOCH₃).

The compounds of formula (I) can easily be prepared by a skilled person using processes and methods well-known in the prior art as e.g. disclosed in WO03066583 which is included explicitly herein by reference.

Preferably, the astaxanthin derivatives with the definition and preferences as given above are used according to the present invention for beautification and improvement of mammalian, in particular human, skin, hair and nails, for providing a moisturizing effect, for providing a deodorizing effect, for prevention and/ or treatment of wrinkles, for prevention and/or treatment of photo-damage, for prevention and/ or treatment of oxidative stress phenomena, for prevention and/ or treatment of cellulite, for prevention and/ or treatment of pigmentation disorders, for thickening of the epidermis, for inhibition and treatment of canities, for rejuvenation of senescent skin cells and/ or for prevention and/ or treatment of inflammatory diseases of the skin. More preferably, the astaxanthin derivatives are used for prevention and/ or treatment of wrinkles, for thickening of the epidermis, for the treatment of inflammatory diseases of the skin and/ or for moisturizing the skin. Even more preferably, the astaxanthin derivatives are used for the prevention and/ or treatment of wrinkles.

In another embodiment the invention relates to the use of an astaxanthin derivative with the definition and preferences as given above as antiageing agent, as anti hyperpigmentation agent, as antioxidant, as anti stress agent, as anti inflammatory agent, as anti-acne agent, as moisturizing agent and/ or as coloring agent, preferably as anti-ageing agent in cosmetic compositions. Preferably, the cosmetic compositions are oral and topical preparations, most preferably topical preparations.

The present invention also relates to compositions, in particular cosmetic compositions for oral or topical application comprising an astaxanthin derivative with the definition and preferences as given. Preferred according to the invention are cosmetic compositions for topical application e.g. in the form of creams, lotions, gels, tonics, oils, powders and shampoos comprising an astaxanthin derivative of formula (I) with the definitions and preferences as given above, and a vehicle! carrier conventionally used in cosmetic compositions.

The amount of the astaxanthin derivative in the cosmetic composition will depend upon the nature of the product and the condition to be treated. However, the cosmetic compositions comprise the astaxanthin derivative in an amount of at least 0.001 wt.-%. Preferably, the astaxanthin derivative of formula (I) is contained in the compositions according to the invention in a concentration of about 0.001 to 20 wt.-%, based on the total weight of the composition. More preferably, the astaxanthin derivative is contained in the composition in an amount of about 0.001 wt% to about 10 wt.-%, in particular in an amount of about 0.01 wt.-% to about 2 wt.-%, such as in an amount of about 0.05 wt.-%, based on the total amount of the composition.

If the topical compositions are intended to be applied to and left on the skin an amount of about 0.0005 to 10 wt.-%, preferably an amount from about 0.001 to 3 wt.-%, most preferably from about 0.001 to 1 wt.-% of an astaxanthin derivative is used based on the total weight of the composition.

If the astaxanthin derivative is to be used in a compositions intended to be applied to, and subsequently rinsed of, the skin or scalp, e.g. in the form of a shampoo, then it is preferred to use the compound in an amount from about 0.01 to 30 wt.%, preferably from about 0.05 to 10 wt.%, in particular from about 0.1 to 5 wt.-% based on the total weight of the composition.

If the astaxanthin is to be used in oral preparations e.g. in form of a capsule or a tablet for oral treatment of the skin conditions as defined above such as for example for the prevention and/ or treatment of wrinkles, then it is preferred to use the compound in an amount from about 0.01 to 10 wt.-%, preferably from about 0.05 to 5 wt.-%, in particular from about 0.1 to 2 wt.-% based on the total weight of the composition.

The cosmetic compositions of the present invention are preferably applied at least once per day, e.g. twice or triple times a day.

The present invention also provides cosmetic compositions comprising an astaxanthin derivative represented by general formula (I) as defined above, and at least one additional ingredient selected from anti-wrinkle/ anti-atrophy/ anti-ageing actives, exfoliating ingredients, anti-oxidants/ radical scavengers, flavonoids, anfi-cellulite agents, tanning actives, skin lightening agents, sunscreen actives, particulate matter, hair growth actives, penetration enhancers/ delivery agents, skin soothing actives, anti-acne actives, fragrances, dyes, preservatives, skin conditioning agents, pigments, emulsifiers and co-emulsifrers. Such compositions comprising an astaxanthin derivative with the definition and preferences as given above and optionally a further active ingredient are novel and are also part of the invention.

Anti-wrinkle/ anti-atrophy/ anti-ageing actives which may be used according to the invention are disclosed e.g. in WO 2004/037213. Exemplary anti-wrinklel anti-atrophy/ anti-ageing actives suitable for use in the compositions of the present invention include sulfur containing D and L amino acids and their derivatives and salts, particularly the N-acetyl derivatives, a preferred example of which is N-acetyl-L-eysteine; thiols, e.g. ethane thiol; hydroxy acids (e.g., alpha-hydroxy acids such as lactic acid and glycolic acid or beta-hydroxy acids such as salicylic acid and salicylic acid derivatives such as the octanoyl derivative), phytic acid, lipoic acid; lysophosphatidic acid, skin peel agents (e.g., phenol and the like), which enhance skin condition and Vitamin A and its derivatives,

Anti-oxidants/ radical scavengers which may be used in compositions according to the invention are known in the art. Especially preferred are antioxidants/ radical scavengers chosen from the group consisting of amino acids (e.g. glycine, histidine, tyrosine, tryptophan) and their derivatives, imidazole (e.g. urocanic acid) and derivatives, peptides such as D,L-carnosine, D-carnosine, L-camosine and derivatives (e.g. anserine), carotenoids, carotenes (e.g. α-carotene, β-carotene, lycopene) and derivatives, chlorogenic acid and derivatives, lipoic acid and derivatives (e.g. dihydrolipoic acid), aurothioglucose, propylthiouracil and other thiols (e.g. thioredoxine, glutathione, cysteine, cystine, cystamine and its glycosyl-, N-acetyl-, methyl-, ethyl-, propyl-, amyl-, butyl- and lauryl-, palmitoyl-; oleyl-, γ-linoleyl-, cholesteryl- and glycerylester) and the salts thereof, dilaurylthiodipropionate, distearylthiodipropionate, thiodipropionic acid and its derivatives (ester, ether, peptides, lipids, nucleotides, nucleosides and salts) as well as sulfoximine compounds (such as buthioninsulfoximine, homocysteinsulfoximine, buthioninsulfone, penta-, hexa-, heptathioninsulfoximine) in very low compatible doses (e.g. pmol to µmol/kg), (metal)-chelators (such as α-hydroxyfatty acids, palmic-, phytinic acid, lactoferrin), β-hydroxyacids (such as citric acid, lactic acid, malic acid), huminic acid, gallic acid, gallic extracts, bilirubin, biliverdin, EDTA, EGTA and its derivatives, unsaturated fatty acids and their derivatives (such as γ-linoleic acid, linolic acid, oleic acid), folic acid and its derivatives, ubiquinone and ubiquinol and their derivatives, vitamin C and derivatives (such as ascorbylpalmitate and ascorbyltetraisopalmitate, Mg-ascorbylphosphate, Naascorbylphosphate, ascorbylacetate), tocopherole and derivates (such as vitamin-E-acetate), mixtures of nat. vitamin E, vitamin A and derivatives (vitamin-A-palmftate and -acetate) as well as coniferylbenzoate, rutinic acid and derivatives, α-glycosylrutin, ferulic acid, furfurylidenglucitol, carnosine, butylhydroxytoluene, butylhydroxyanisole, trihydroxybutyrophenone, urea and its derivatives, mannose and derivatives, zinc and derivatives (e.g. ZnO, ZnSO₄), selenium and derivatives (e.g. selenomethionin), stilbenes and derivatives (such as stilbenoxide, trans-stilbenoxide). Suitable derivatives of these active ingredients are e.g. salts, esters, ethers, sugars, nucleotides, nucleosides, peptides and lipids. One or more antioxidants/radical scavengers may be present in an amount about 0.01 wt.-% to about 10 wt.% of the total weight of the composition of the present invention. Preferably, one or more antioxidants/radical scavengers are present in an amount about 0.1 wt-% to about 1 wt.%.

Suitable flavonoids which may be used in compositions according to the invention are disclosed e.g. in WO 2004/037213 and in U.S. patents 5,686,082 and 5,686,367, all of which are herein incorporated by reference. Flavonoids suitable for use in the present invention are flavanones selected from unsubstituted flavanones, mono-substituted flavanones, and mixtures thereof; chalcones selected from unsubstituted chalcones, mono-substituted chalcones, di-substituted chalcones, tri-substituted chalcones, and mixtures thereof; flavones selected from unsubstituted flavones, mono-substituted flavones, di-substituted flavones, and mixtures thereof; one or more isoflavones; coumarins selected from unsubstituted coumarins, mono-substituted coumarins, di-substituted coumarins, and mixtures thereof; chromones selected from unsubstituted chromones, mono-substituted chromones, di-substituted chromones, and mixtures thereof; one or more dicoumarols; one or more chromanones; one or more chromanols; isomers (e.g., cis/trans isomers) thereof; and mixtures thereof. The term "substituted" as used herein means flavonoids wherein one or more hydrogen atoms of the flavonoid has been independently replaced with hydroxyl, C₁-C₈-alkyl. C₁-C₄-alkoxyl, O-glycoside, and the like or a mixture of these substituents.

Anti-cellulite agents which may be incorporated into compositions according to the invention are known in the art and include, but are not limited to, xanthine compounds (e.g. caffeine, theophylline, theobromine and aminophylline).

Tanning actives are known in the art. If a tanning active is included with the compositions of the present invention, it is preferably present from approximately 0.1 wt.-% to 20 wt.-%. An example of a suitable tanning active is dihydroxyacetone.

Suitable skin lightening agents are disclosed e.g. in WO 2004/037213. When present, the compositions preferably contain from about 0.1 wt.-% to about 10 wt.-%, more preferably from about 0.2 wt.-% to about 5 wt.-%, also preferably from about 0.5 wt.-% to about 2 wt.-% of a skin lightening agent. Suitable skin lightening agents include those known in the art, including kojic acid, arbutin, ascorbic acid and derivatives thereof (e.g., magnesium ascorbyl phosphate or sodium ascorbyl phosphate), and extracts (e.g., mulberry extract, placental extract). Skin lightening agents suitable for use herein also include those described in WO 95134280 and WO 95/23780.

Suitable sunscreen actives are disclosed e.g. in WO 2004/037213. As used herein, "sunscreen active" includes both sunscreen agents and physical sunblocks. Suitable sunscreen actives may be organic or inorganic. Inorganic sunscreens useful herein include metallic oxides, in particular titanium dioxide having an average primary particle size of from about 15 nm to about 100 nm, zinc oxide having an average primary particle size of from about 15 nm to about 150 nm, zirconium oxide having an average primary particle size of from about 15 nm to about 150 nm, iron oxide having an average primary particle size of from about 15 nm to about 500 nm, and mixtures thereof. When used herein, the inorganic sunscreens are present in the amount of from about 0.1% to about 20%, preferably from about 0.5% to about 10%, more preferably from about 1% to about 5%, by weight of the composition. A wide variety of conventional organic sunscreen actives are suitable for use herein. Sagarin, et al., at Chapter VIII, pages 189 et seq., of Cosmetics Science and Technology (1972), discloses numerous suitable actives. Specific suitable sunscreen actives include, for example: p-aminobenzoic acid, its salts and its derivatives (ethyl, isobutyl, glyceryl esters; p-dimethylaminobenzoic acid); anthranilates (i.e., o-aminobenzoates; methyl, menthyl, phenyl, benzyl, phenylethyl, linalyl, terpinyl, and cyclohexenyl esters); salicylates (amyl, phenyl, octyl, benzyl, menthyl, glyceryl, and di-propylene glycol esters); cinnamic acid derivatives (menthyl and benzyl esters, α-phenyl cinnamonitrile; butyl cinnamoyl pyruvate); dihydroxycinnamic acid derivatives (umbelliferone, methylumbelliferone, methylaceto-umbelliferone); trihydroxy-cinnamic acid derivatives (esculetin, methylesculetin, daphnetin, and the glucosides, esculin and daphnin); hydrocarbons (diphenylbutadiene, stilbene); dibenzalacetone and benzalacetophenone; naphtholsulfonates (sodium salts of 2-naphthol-3,6-disulfonic and of 2-naphthol-6,8-disulfonic acids); di-hydroxynaphthoic acid and its salts; o- and p-hydroxybiphenyldisulfonates; coumarin derivatives (7-hydroxy, 7-methyl, 3-phenyl); diazoles (2-acetyl-3-bromoindazole, phenyl benzoxazole, methyl naphthoxazole, various aryl benzothiazoles); quinine salts (bisulfate, sulfate, chloride, oleate, and tannate); quinoline derivatives (8-hydroxyquinoline salts, 2-phenylquinoline); hydroxy- or methoxy-substituted benzophenones; uric and violuric acids; tannic acid and its derivatives (e.g., hexaethylether); (butyl carbotol) (6-propyl piperonyl) ether, hydroquinone; benzophenones (oxybenzene, sulisobenzone, dioxybenzone, benzoresorcinol, 2,2',4,4'-tetrahydroxybenzophenone, 2,2'-dihydroxy-4,4'-dimethoxybenzophenone, octabenzone; 4-isopropyldibenzoylmethane; butylmethoxydibenzoylmethane; etocrylene; octocrylene; [3-(4'-methylbenzylidene boman-2-one), terephthalylidene dicamphor sulfonic acid and 4-isopropyl-di-benzoylmethane.

Also particularly useful in the compositions are sunscreen actives such as those disclosed in U.S. patent No. 4,937,370 and U.S. patent No. 4,999,186. The sunscreen agents disclosed therein have, in a single molecule, two distinct chromophore moieties which exhibit different ultra-violet radiation absorption spectra. One of the chromophore moieties absorbs predominantly in the UVB radiation range and the other absorbs strongly in the UVA radiation range. A safe and effective amount of the organic sunscreen active is used, typically from about 1% to about 20%, more typically from about 2% to about 10% by weight of the composition. Exact amounts will vary depending upon the sunscreen or sunscreens chosen and the desired Sun Protection Factor (SPF).

Particularly preferred sunscreens are sunscreens on the basis of polysiloxanes such as Parsol SLX, described e.g. in EP 358 584, EP 538 431 and EP 709 080.

Suitable particulate matter is disclosed in WO 2004/037213. These particulates can be coated or uncoated, charged or uncharged. Charged particulate materials are disclosed in U.S. patent No. 5,997,887. Particulate materials useful herein include: bismuth oxychloride, iron oxide, mica, mica treated with barium sulfate and TiO₂, silica, nylon, polyethylene, talc, styrene, polypropylene, ethylene/acrylic acid copolymer, titanium dioxide iron oxide, bismuth oxychloride, sericite, aluminum oxide, silicone resin, barium sulfate, calcium carbonate, cellulose acetate, polymethyl methacrylate, and mixtures thereof.

Examples of penetration enhancers/ delivery agents include in particular 2-methyl propan-2-ol, propan-2-ol, ethyl-2-hydroxypropanoate, hexan-2,5-diol, POE(2) ethyl ether, di(2-hydroxypropyl) ether, pentan-2,4-diol, acetone, POE(2) methyl ether, 2-hydroxypropionic acid, 2-hydroxyoctanoic acid, propan-1-ol, 1,4-dioxane, tetrahydrofuran, butan-1,4-diol, propylene glycol dipelargonate, polyoxypropylene 15 stearyl ether, octyl alcohol, POE ester of oleyl alcohol, oleyl alcohol, lauryl alcohol, dioctyl adipate, dicapryl adipate, diisopropyl adipate, diisopropyl sebacate, dibutyl sebacate, diethyl sebacate, dimethyl sebacate, dioctyl sebacate, dibutyl suberate, dioctyl azelyate, dibenzyl sebacate, dibutyl phthalate, dibutyl azelate, ethyl myristate, dimethyl azelate, butyl myristate, dibutyl succinate, didecyl phthalate, decyl oleate, ethyl caproate, ethyl salicylate, isopropyl palmitate, ethyl laurate, 2-ethyl-hexyl pelargonate, isopropyl isostearate, butyl laurate, benzyl benzoate, butyl benzoate, hexyl laurate, ethyl caprate, ethyl caprylate, butyl stearate, benzyl salicylate, 2-hydroxypropanoic acid, 2-hydroxyoctanoic acid, dimethyl sulphoxide, N,N-dimethyl acetamide, N,N-dimethyl formamide, 2-pyrrolidone, 1-methyl-2-pyrrolidone, 5-methyl-2-pyrrolidone, 1,5-dimethyl-2-pyrrolidone, 1-ethyl-2-pyrrolidone, phosphine oxides, sugar esters, tetrahydrofurfural alcohol, urea, diethyl-m-toluamide, and 1-dodecyiazacycloheptan-2-one.

Skin soothing actives which may be incorporated into compositions according to the invention include panthenoic acid derivatives (including panthenol, dexpanthenol, ethyl panthenol), aloe vera, allantoin, bisabolol and dipotassium glycyrrhizinate. Anti-acne actives include Vitamin C and its derivatives.

If nothing else is stated, the additional active ingredient is present in the compositions of the present invention in an amount of preferably 0.1 to 20, more preferably of 1 to 10 wt.-%, based on the weight of the composition.

The term "cosmetic preparation" or "cosmetic composition" as used in the present specification refers to cosmetic compositions as defined under the heading "Kosmetika" in R6mpp Lexikon Chemie, 10th edition 1997, Georg Thieme Verlag Stuttgart, New York.

Furthermore, the term "cosmetic composition" or "cosmetic preparation" includes compositions for oral application which have a cosmetic effect.

The cosmetic compositions of the present invention contain the astaxanthin derivative with the definitions and preferences as given above together with cosmetically acceptable excipients or diluents.

Regarding the kind of the topical preparation and the manufacture of the topical preparations as well as for further suitable additives, it can be referred to the pertinent literature, e.g. to Novak G.A., Die kosmetischen Präparate - Band 2, Die kosmetischen Präparate - Rezeptur, Rohstoffe, wissenschaftliche Grundiagen (Verlag für Chem. Industrie H. Ziolkowski KG, Augsburg).

If nothing else is stated, in this specification parts and percentages are per weight and are based on the weight of the composition.

Preferably, the compositions of the present invention are topical preparations, such as liquid or solid oil-in-water emulsions, water-in-oil emulsions, multiple emulsions, microemulsions, PET-emulsions, bickering emulsions, hydrogels, alcoholic gels, lipogels, one or multiphase solutions, foams, ointments, plasters, suspensions, powders, creams, cleanser, soaps and other usual compositions, which can also be applied by pens, as masks or as sprays.

More preferably, the compositions of the present invention are topical preparations selected from the group consisting of liquid or solid oil-in-water emulsions, water-in-oil emulsions, multiple emulsions, microemulsions, PET-emulsions, bickering emulsions, hydrogels, alcoholic gels, lipogels, one or multiphase solutions, foams, creams, cleanser and soaps.

In another preferred embodiment the composition according to the invention are decorative preparations such as lipsticks, nail varnishes, eye shadow, mascaras, dry and moist makeup, rouge, powders, depilatory agents, and suntan lotions.

The topical preparations of the present invention usually contain cosmetic adjuvants and additives, such as fatty substances/ oils, water, organic solvents, silicones, thickeners, softeners, emulsifiers, antifoaming agents, moisturizers, surfactants, fillers, sequestering agents, anionic, cationic, nonionic or amphoteric polymers or mixtures thereof, propellants, acidifying or basifying agents, or any other ingredients usually formulated into cosmetics.

Typically topical preparations also contain surface active ingredients like emulsifiers, solubilizers and the like. An emulsifier enables two or more immiscible components to be combined homogeneously. Moreover, the emulsifier acts to stabilize the composition. Emulsifiers that may be used in the present invention in order to form O/W, W/O, O/W/O or W/O/W emulsions/microemulsions include sorbitan oleate, sorbitan sesquioleate, sorbitan isostearate, sorbitan trioleate, polyglyceryl-3-diisostearate, polyglycerol esters of oleic/isostearic acid, polyglyceryl-6 hexaricinolate, polyglyceryl-4-oleate, polygylceryl-4 oleate/PEG-8 propylene glycol cocoate, oleamide DEA, TEA myristate, TEA stearate, magnesium stearate, sodium stearate, potassium laurate, potassium ricinoleate, sodium cocoate, sodium tallowate, potassium castorate, sodium oleate, and mixtures thereof. Further suitable emulsifiers are phosphate esters and the salts thereof such as cetyl phosphate (Amphisol®A), diethanolamine cetyl phosphate (Amphisol®), potassium cetyl phosphate (Amphisol®K), sodium glyceryl oleate phosphate, hydrogenated vegetable glycerides phosphate and mixtures thereof. Furthermore, one or more synthetic polymers may be used as an emulsifier. For example, PVP eicosene copolymer, acrylates/C₁₀₋₃₀ alkyl acrylate crosspolymer, acrylateslsteareth-20 methacrylate copolymer, PEG-22/dodecyl glycol copolymer, PEG-45/dodecyl glycol copolymer and mixtures thereof. The preferred emulsifiers are cetyl phosphate (Amphisol® A), diethanolamine cetyl phosphate (Amphisol®), potassium cetyl phosphate (Amphisol® K), PVP Eicosene copolymer, acrylates/C₁₀₋₃₀-alkyl acrylate crosspolymer, PEG-20 sorbitan isostearate, sorbitan isostearate, and mixtures thereof. The one or more emulsifiers are present in a total amount about 0.01 wt.-% to about 20 wt.-% of the total weight of the composition of the present invention. Preferably, about 0.1 wt.-% to about 10 wt.-% of emulsifiers are used.

The lipid phase of the topical preparations can advantageously be chosen from:
- mineral oils and mineral waxes;
- oils such as triglycerides of caprinic acid or caprylic acid, preferable castor oil;
- oils or waxes and other natural or synthetic oils, in an preferred embodiment esters of fatty acids with alcohols e.g. isopropanol, propylene glycol, glycerin or esters of fatty alcohols with carbonic acids or fatty acids;
- alkylbenzoates; and/or
- silicone oils such as dimethylpolysiloxane, diethylpolysiloxane, diphenylpolysiloxane, cyclomethicones
and mixtures thereof. Preferably, however, the lipid phase does not contain a mineral oil.

Exemplary fatty substances which can be incorporated in the oil phase of the emulsion, microemulsion, oleo gel, hydrodispersion or lipodispersion of the present invention are advantageously chosen from esters of saturated and/ or unsaturated, linear or branched alkyl carboxylic acids with 3 to 30 carbon atoms, and saturated and/ or unsaturated, linear and/ or branched alcohols with 3 to 30 carbon atoms as well as esters of aromatic carboxylic acids and of saturated and/ or unsaturated, linear or branched alcohols of 3-30 carbon atoms. Such esters can advantageously be selected from octylpalmitate, octylcocoate, octylisostearate, octyldodecylmyristate, cetearylisononanoate, isopropylmyristate, isopropylpalmitate, isopropylstearate, isopropyloleate, n-butylstearate, n-hexylleureate, n-decytoleat, isooctylstearate, isononylstearate, isononylisononanoate, 2-ethyl hexylpalmitate, 2-ethylhexyllaurate, 2-hexyldecylstearate, 2-octyldodecylpalmitate, stearylheptanoate, oleyloleate, oleylerucate, erucyloleate, erucylerucate, tridecytstearate, tridecyltrimellitate, as well as synthetic, half-synthetic or natural mixtures of such esters e.g. jojoba oil.

Other fatty components suitable for use in the topical preparations of the present invention include polar oils such as lecithins and fatty acid triglycerides, namely triglycerol esters of saturated and/or unsaturated, straight or branched carboxylic acid with 8 to 24 carbon atoms, preferably of 12 to 18 carbon-atoms whereas the fatty acid triglycerides are preferably chosen from synthetic, half synthetic or natural oils (e.g., cocoglyceride, olive oil, sun flower oil, soybean oil, peanut oil, rape seed oil, sweet almond oil, palm oil, coconut oil, castor oil, hydrogenated castor oil, wheat oil, grape seed oil, macadamia nut oil and others); apolar oils such as linear and/ or branched hydrocarbons and waxes e.g. mineral oils, vaseline (petrolatum); paraffins, squalane and squalene, polyolefins, hydrogenated polyisobutenes and isohexadecanes, favored polyolefins are polydecenes; dialkyl ethers such as dicaprylylether; linear or cyclic silicone oils such as preferably cyclomethicone (octamethylcyclotetrasiloxane; cetyldimethicone, hexamethylcyclotrisiloxane, polydimethylsiloxane, poly(methylphenylsiloxane) and mixtures thereof.

Other fatty components which can advantageously be incorporated in topical preparations of the present invention are isoeikosane; neopentylglycoldiheptanoate; propyleneglycoldicaprylate/ dicaprate; caprylic/ capric/ diglycerylsuccinate; butyleneglycol caprylat/caprat; C₁₂-₁₃-alkyllactate; di-C₁₂-₁₃ alkyltartrate; triisostearin; dipentaerythrityl hexacaprylat/hexacaprate; propyleneglycolmonoisostearate; tricaprylin; dimethylisosorbid. Especially beneficial is the use of mixtures C₁₂₋₁₅-alkylbenzoate and 2-ethylhexylisostearate, mixtures C₁₂₋₁₅-alkylbenzoate and isotridecylisononanoate as well as mixtures of C₁₂₋₁₅-alkylbenzoate, 2-ethylhexylisostearate and isotridecylisononanoate.

The oily phase of the compositions of the present invention can also contain natural vegetable or animal waxes such as bee wax, china wax, bumblebee wax and other waxes of insects as well as shea butter and cocoa butter.

A moisturizing agent may be incorporated into a topical preparation of the present invention to maintain hydration or rehydrate the skin. Moisturizers that prevent water from evaporating from the skin by providing a protective coating are called emollients. Additionatly an emollient provides a softening or soothing effect on the skin surface and is generally considered safe for topical use, Preferred emollients include mineral oils, lanolin, petrolatum, capric/caprylic triglyceraldehydes, cholesterol, silicones such as dimeticone, cyclometicone, almond oil, jojoba oil, avocado oil, castor oil, sesame oil, sunflower oil, coconut oil and grape seed oil, cocoa butter, olive oil aloe extracts, fatty acids such as oleic and stearic, fatty alcohols such as cetyl and hexadecyl (ENJAY), diisopropyl adipate, hydroxybenzoate esters, benzoic acid esters of C₉₋₁₅-alcohols, isononyl iso-nonanoate, ethers such as polyoxypropylene butyl ethers and polyoxypropylene cetyl ethers, and C₁₂₋₁₅- alkyl benzoates, and mixtures thereof. The most preferred emollients are hydroxybenzoate esters, aloe vera, C₁₂₋₁₅-alkyl benzoates, and mixtures thereof. An emollient is present in an amount of about 1 wt.-% to about 20 wt.-% of the total weight of the composition. The preferred amount of emollient is about 2 wt.-% to about 15 wt.-%, and most preferably about 4 wt.-% to about 10 wt.-%.

Moisturizers that bind water, thereby retaining it on the skin surface are called humectants. Suitable humectants can be incorporated into a topical preparation of the present invention such as glycerin, polypropylene glycol, polyethylene glycol, lactic acid, pyrrolidone carboxylic acid, urea, phospholipids, collagen, elastin, ceramides, lecithin sorbitol, PEG-4, and mixtures thereof. Additional suitable moisturizers are polymeric moisturizers of the family of water soluble and/ or swellable/ and/ or with water gelating polysaccharides such as hyaluronic acid, chitosan and/or a fucose rich polysaccharide which is e.g. available as Fucogel®1000 (CAS-Nr. 178463-23-5) by SOLABIA S. One or more humectants are optionally present at about 0.5 wt.-% to about 8 wt.-% in a composition of the present invention, preferably about 1 wt.-% to about 5 wt.-%.

The aqueous phase of the preferred topical preparations of the present invention can contain the usual cosmetic additives such as alcohols, especially lower alcohols, preferably ethanol and/ or isopropanol, low diols or polyols and their ethers, preferably propyleneglycol, glycerin, ethyleneglycol, ethyleneglycol monoethyl- or monobutylether, propyleneglycol monomethyl- or -monoethyl- or -monobutylether, diethyleneglycol monomethyl-or monoethylether and analogue products, polymers, foam stabilizers; electrolytes and especially one or more thickeners. Thickeners that may be used in formulations of the present invention to assist in making the consistency of a product suitable include carbomer, siliciumdioxide, magnesium and/ or aluminum silicates, beeswax, stearic acid, stearyl alcohol polysaccharides and their derivatives such as xanthan gum, hydroxypropyl cellulose, polyacrylamides, acrylate crosspolymers preferably a carbomer, such as carbopole® of type 980, 981, 1382, 2984, 5984 alone or mixtures thereof. Suitable neutralizing agents which may be included in the composition of the present invention to neutralize components such as e.g. an emulsifier or a foam builder/stabilizer include but are not limited to alkali hydroxides such as a sodium and potassium hydroxide; organic bases such as diethanolamine (DEA), triethanolamine (TEA), aminomethyl propanol, and mixtures thereof; amino acids such as arginine and lysine and any combination of any foregoing. The neutralizing agent can be present in an amount of about 0.01 wt.-% to about 8 wt.-% in the composition of the present invention, preferably, 1 wt.-% to about 5 wt.-%.

The addition of electrolytes into the composition of the present invention may be necessary to change the behavior of a hydrophobic emulsifier. Thus, the emulsions/microemulsions of this invention may contain preferably electrolytes of one or several salts including anions such as chloride, sulfates, carbonate, borate and aluminate, without being limited thereto. Other suitable electrolytes can be on the basis of organic anions such as, but not limited to, lactate, acetate, benzoate, propionate, tartrate and citrate. As cations preferably ammonium, alkylammonium, alkali- or alkaline earth metals, magnesium-, iron- or zinc-ions are selected. Especially preferred salts are potassium and sodium chloride, magnesium sulfate, zinc sulfate and mixtures thereof. Electrolytes can be present in an amount of about 0.01 wt.-% to about 8 wt.-% in the composition of the present invention.

The topical preparations of the present invention can preferably be provided, in the form of a lotion, a thickened lotion, a gel, a cream, a milk, an ointment, a shampoo, a powder or a solid tube stick and can be optionally be packaged as an aerosol and can be provided in the form of a mousse, foam or a spray. The compositions according to the invention can also be in the form of a suspension or dispersion in solvents or fatty substances, or alternatively in the form of an emulsion or microemulsion (in particular of O/W or W/O type, O/W/O or W/O/W-type), such as a cream or a milk, a vesicular dispersion, in the form of an ointment, a gel, a solid tube stick or an aerosol mousse. The emulsions can also contain anionic, nonionic, cationic or amphoteric surfactants.

The topical application is preferably at least once per day, e.g. two or three times a day. Usually it takes at least two weeks until the desired effect starts to show up and some more weeks until the maximum benefit is achieved. However, it can also take several weeks or even months until the desired effect is achieved. In some cases it might be advisable to perform the treatment in intervals to maximize the benefit.

The amount of the topical preparation which is to be applied to the skin depends on the concentration of the active ingredients in the compositions and the desired cosmetic or pharmaceutical effect. For example, application can be such that a cream is applied to the skin. A cream is usually applied in an amount of 2 mg cream/cm² skin. The amount of the composition which is applied to the skin is, however, not critical, and if with a certain amount of applied composition the desired effect cannot be achieved, a higher concentration of the active ingredients can be used e.g. by applying more of the composition or by applying compositions which contain more active ingredient.

According to the invention for preparing the compositions the active ingredients can be used as such or in an encapsulated form, for example in a liposomal form. Liposomes are preferably formed with lecithins with or without addition of sterols or phytosterols. The encapsulation of the active ingredients can be alone or together with other active ingredients.

If the composition according to the invention is intended for oral application, the oral preparation may be in the form of a liquid, gel, gelcap, capsule, powder, solid tablet (coated or non-coated) or the like, preferably in the form of a tablet or capsule or a hard (shell) gelatin capsule. Suitable excipient and/or carriers for oral application include maltodextrin, calcium carbonate, dicalcium phosphate, tricalcium phosphate, microcrystalline cellulose, dextrose, rice flour, magnesium stearate, stearic acid, croscarmellose sodium, sodium starch glycolate, crospovidone, sucrose, vegetable gums, lactose, methylcellulose, povidone, carboxymethylcellulose, com starch, and the like (including mixtures thereof). Preferred carriers include calcium carbonate, magnesium stearate, maltodextrin, and mixtures thereof. The various ingredients and the excipient and/or carrier are mixed and formed into the desired form using conventional techniques. The tablet or capsule of the present invention may be coated with an enteric coating that dissolves at a pH of about 6.0 to 7.0. A suitable enteric coating that dissolves in the small intestine but not in the stomach is cellulose acetate phthalate. Further details on techniques for formulation for and administration may be found in the latest edition of Remington's Pharmaceutical Sciences (Maack Publishing Co., Easton, PA).

The following examples further illustrate the invention, but they should not be construed as limiting the invention.

### Example 1: Facial anti-wrinkle treatment formulations

| Phase | *INCl Nomenclature* | % *w* / *w* |
|---|---|---|
| 1 | Glyceryl Myristate | 5.00 |
| | Cetyl Alcohol | 2.00 |
| | Cetyl Phosphate | 2.00 |
| | Isopropyl Myristate | 10.00 |
| | Tocopheryl Acetate | 0.30 |
| | Prunus Amygdalus Dulcis (Sweet Almond) Oil | 2.00 |
| | Phenoxyethanol & Methylparaben & Ethyfparaben & Propylparaben & Butylparaben & Isopropylparabon | 0.60 |
| | (all-E)-3,3'-astaxanthin-dimethyldisuccinate | 0.001 |
| | | |
| 2 | Water | Ad. 100 |
| | D-Panthenol | 0.20 |
| | Disodium EDTA | 0.10 |
| | Propylene Glycol | 4.00 |
| | Polyacrylamide & C13-14 Isoparaffin & Laureth-7 | 2.00 |
| | | |
| 3 | Sodium hydroxide 10 % | q.s. |

Procedure: Heat part 1 and 2 separately to 80°C temperature, let both cool to 60°C and add the astaxanthin derivative to part 1. Add part 2 into part 1 under agitation and homogenize. Cool during agitation to ambient tempoerature. Adjust with part 3 to pH 6.5-7.5. It is generally recommended to use vacuum while producing the emulsion.

### Example 2: Suncream

| Phase | *INCI Nomenclature* | % *w* / *w* |
|---|---|---|
| 1 | Polysilicone-15 | 5.00 |
| | Octocrylene | 3.00 |
| | Ethylhexyl Methoxycinnamate | 6.00 |
| | PPG15 Stearyl Ether | 6.00 |
| | Isopropyl Palmitate | 4.00 |
| | Dimethicone | 2.00 |
| | Steareth-2 | 2.00 |
| | Steareth-21 | 2.00 |
| | Cetyl Alcohol | 2.00 |
| | Stearyl Alcohol | 1.00 |
| | Behenyl Alcohol | 1.00 |
| | BHT | 0.05 |
| | Phenoxyethanol & Methylparaben & Ethylparaben & Butylparaben & Propylparaben & Isobutylparaben | 0.60 |
| | (all-E)-3,3'-astaxanthin-dimethyldisuccinate | 0.008 |
| | | |
| 2 | Glycerin | 5.00 |
| | Disodium EDTA | 0.10 |
| | Aqua | Ad 100 |
| | | |
| 3 | Potassium Hydroxide | q.s. |

Procedure: Heat part 1 and 2 separately to 80°C temperature, let both cool to 60°C and add the astaxanthin derivative to part 1. Add part 2 into part 1 under agitation and homogenize. Cool during agitation to ambient temperature. Adjust with part 3 to pH 6.5-7.5. It is generally recommended to use vacuum while producing the emulsion.

### Example 3: Sunscreen

| Phase | *INCl Nomenclature* | % *w* / *w* |
|---|---|---|
| 1 | Ethylhexyl Methoxycinnamate | 6.00 |
| | Butyl Methoxydibenzoylmethane | 3.00 |
| | 4-Methylbenzylidene Camphor | 1.80 |
| | Bis-Stearyl Dimethicone | 2.00 |
| | Caprylic/Capric Triglyceride | 7.00 |
| | Cetyl Alcohol | 1.00 |
| | Glyceryl Myristate | 3.00 |
| | BHT | 0.05 |
| | Phenoxyethanol & Methylparaben & Ethylparaben & Butylparaben & Propylparaben & lsobutylparaben | 0.60 |
| | Cetyl Phosphate | 2.00 |
| | (all-E)-3,3'-astaxanthin-dimethyldisuccinate | 0.005 |
| | | |
| 2 | Carbomer | 0.20 |
| | Glycerin | 3.00 |
| | Disodium EDTA | 0.10 |
| | Aqua | Ad 100 |
| | | |
| 3 | Tromethamine | 1.00 |

Procedure: Heat part 1 and part 2 separately to 80°C, let both cool to 60°C and add the astaxanthin derivative to part 1. Add part 2 into part 1 under agitation and homogenize. Cool during agitation to ambient temperature. Adjust with part 3 to pH 7.0 - 7.5. It is generally recommended to use vacuum while producing the emulsion.

### Example 4 : Moisturizing Day Cream

| Phase | *INCI Nomenclature* | *% w l w* |
|---|---|---|
| 1 | Glyceryl Stearate & Ceteareth-20& Ceteareth-12 & Cetearyl Alcohol & Cetyl Palmitate | 8.00 |
| | lsopropyl Myristate | 6.00 |
| | Propylene Glycol Dicaprylate/Dicaprate | 6.00 |
| | Tocopheryl Acetate | 2.00 |
| | Phenoxyethanol & Methylparaben & Ethylparaben & Butylparaben & Propylparaben & Isobutylparaben | 0.80 |
| | (all-E)-3,3'-astaxanthin-dimethyldisuccinate | 0.001 |
| | | |
| 2 | Glycerin | 3.00 |
| | Disodium EDTA | 0.10 |
| | Panthenol | 2.70 |
| | Aqua | Ad 100 |
| | | |
| 3 | Polyacrylamide & C13-14 Isoparaffin & Laureth-7 | 3.00 |

Procedure: Heat part 1 up to 85°C; and heat also part 2 up to 85°C, let both cool to 60°C and add the astaxanthin derivative to part 1. When both have the same temperature, add 2 to 1 while homogenizing intensively. Cool down the product to 45°C while stirring. Now add part 3 and homogenize intensively again. Cool down the emulsion to ambient temperature while stirring.
It is generally recommended to use vacuum while producing the emulsion.

### Example 5 : Anti age spot cream

| Phase | *INCl Nomenclature* | % *w* / *w* |
|---|---|---|
| 1 | Glyceryl Myristate | 2.50 |
| | Cetyl Alcohol | 2.50 |
| | Ethylhexyl Methoxycinnamate | 5.00 |
| | Butyl Methoxydibenzoylmethane | 2.00 |
| | Octocrylene | 1.70 |
| | Prunus Amygdalus Dulcis (Sweet Almond) Oil | 2.00 |
| | Diisopropyl Sebacate | 4.00 |
| | BHT | 0.05 |
| | Dimethicone | 0.50 |
| | Tocopheryl Acetate | 1.00 |
| | Phenoxyethanol & Methylparaben & Ethylparaben & Butylparaben & | 0.20 |
| | Propylparaben & Isobutylparaben | |
| | Steareth-2 | 2.00 |
| | Steareth-21 | 2.00 |
| | Cyclomethicone | 4.00 |
| | (all-E)-3,3'-astaxanthin-dimethyidisuccinate | 0.01 |
| | | |
| 2 | Butylene Glycol | 2.00 |
| | Glycerin | 3.00 |
| | Disodium EDTA | 0.10 |
| | Xanthan Gum | 0.20 |
| | Sodium Metabisulfite | 0.05 |
| | AcrylateslC10-30 Alkyl Acrylate Crosspolymer | 0.25 |
| | Aqua | Ad 100 |

Procedure: Melt all components of Part 1 in a vessel at 85°C, let it cool to 60°C and add the astaxanthin derivative to part 1. Heat part 2 up to 85°C, let it cool to 60°C. When both have the same temperature, add part 2 into part 1 under agitation and homogenization. Cool down the emulsion to ambient temperature while stirring. It is generally recommended to use vacuum while producing the emulsion.

### Example 6 : Anti wrinkle cream

| Phase | *INCl Nomenclature* | % *w* / *w* |
|---|---|---|
| 1 | Glyceryl Myristate | 2.50 |
| | Cetyl Alcohol | 2.50 |
| | Ethylhexyl Methoxycinnamate | 5.00 |
| | Butyl Methoxydibenzoylmethane | 2.00 |
| | Octocrylene | 1.70 |
| | Macadamia Ternifolia Seed Oil | 2.00 |
| | C12-15 Alkyl Benzoate | 5.50 |
| | BHT | 0.05 |
| | Dimethicone | 0.50 |
| | Tocopheryl Acetate | 2.00 |
| | Phenoxyethanol | 0.5 |
| | Steareth-2 | 2.00 |
| | Steareth-21 | 2.00 |
| | (all-E)-3,3'-astaxanthin-dimethyldisuccinate | 0.005 |
| | | |
| 2 | Glycerin | 3.00 |
| | Disodium EDTA | 4.14 |
| | Xanthan Gum | 0.20 |
| | Acrylates/C10-30 Alkyl Acrylate Crosspolymer | 0.25 |
| | Aqua | Ad 100 |
| | | |
| 4 | Tromethamine | 0.15 |

Procedure: Heat part 1 up to 85°C, let it cool to 60°C and add the astaxanthin derivative to part 1; and heat also part 2 up to 85°C and let it cool to 60°C. When both have the same temperature add part 2 to part 1 while homogenizing intensively. Cool down the product to 35°C while stirring. Now add part 4 homogenize intensively again. Cool down the emulsion to ambient temperature while stirring.
It is generally recommended to use vacuum while producing the emulsion.

### Example 7 : Anti wrinkle and moisturizing gel

| Phase | *INCI Nomenclature* | *% w l w* |
|---|---|---|
| 1 | Hydroxyethylcellulose | 1.20 |
| | Aqua | Ad 100 |
| | | |
| 2 | Aqua | 2.00 |
| | Panthenol | 1.25 |
| | | |
| 3 | Propylene Glycol | 5.00 |
| | Alcohol | 3.00 |
| | PEG/PPG-20/6 Dimethicone | 0.50 |
| | | |
| 4 | Alcohol | 7.00 |
| | PEG-40 Hydrogenated Castor Oil | 0.80 |
| | (all-E)-3,3'-astaxanthin-dimethyldisuccinate | 0.001 |
| | Parfum | 0.40 |
| | | |
| 5 | Citric Acid | 0.50 |

Procedure: Dissolve Natrosol in Water. Add part 2 under slow agitation to part 1. Add part 3 under slow agitation. Mix all the ingredients of part 4 and add slowly under slow agitation Adjust the pH with part 5.

### Example 8 : Lipstick

| Phase | *INCl Nomenclature* | *% w* / *w* |
|---|---|---|
| 1 | Euphorbia Cerifera (Candelilla) Wax | Ad 100 |
| | Castor Oil | 18.00 |
| | Oley Alcohol | 18.00 |
| | Propylene Glycol Myristate | 15.00 |
| | Mineral Oil | 15.00 |
| | Petrolatum | 10.00 |
| | BHT | 0.02 |
| | | |
| 2 | (all-E)-3,3'-astaxanthin-dimethyldisuccinate | 0.01 |
| | Pigment | 5.00 |
| | | |
| 3 | Fragrance | 0.50 |

Procedure: Heat part 1 to 85°C while stirring, until complete dissolution. Let the mixture cool down to 60°C then add part 2 while mixing. Cool to ambient temperature. Then pass the preparation three times through a three roller mill. Heat again to 75°C, add part 3 while mixing. Pour the hot preparation into jars.

### Example 9 : Lipstick with sunscreens

| Phase | *INCl Nomenclature* | % *w l w* |
|---|---|---|
| 1 | Ethylhexyl Methoxycinnamate | 7.50 |
| | Butyl Methoxydibenzoylmethane | 2.00 |
| | 4-Methylbenzylidene Camphor | 3.00 |
| | Oley Alcohol | 20.00 |
| | Castor Oil | 12.00 |
| | Hydrogenated Castor Oil | 3.00 |
| | Microcrystalline Wax | Ad 100 |
| | Mineral Oil | 5.96 |
| | Petrolatum | 10.00 |
| | Dimethicone | 3.50 |
| | Propylene Glycol | 3.00 |
| | BHT | 0.02 |
| | | |
| | 2 (all-E)-3,3'-astaxanthin-dimethyldisuccinate | 0.05 |
| | Pigment | 5.00 |
| | | |
| | 3 Fragrance | 0.40 |

Procedure: Heat part 1 to 85°C while stirring, until complete dissolution. Let the mixture cool down to 60°C then add part 2 while mixing. Cool to ambient temperature. Then pass the preparation three times through a three roller mill. Heat again to 75°C, add part 3 while mixing. Pour the hot preparation into jars.

### Example 10 : Liptone

| Phase | *INCI Nomenclature* | *% w* / *w* |
|---|---|---|
| 1 | Isostearyl Neopentanoate | 6.00 |
| | Cyclomethicone | 5.00 |
| | Castor Oil | Ad 100 |
| | Coco-Caprylate/Caprate | 15.00 |
| | Tocopheryl Acetate | 2.00 |
| | Euphorbia Cerifera (Candelilla) Wax | 5.00 |
| | Beeswax | 7.50 |
| | Dimethicone Copolyol | 4.00 |
| | Polydecene | 8.00 |
| | Copemicia Cerifera (Camauba) Wax | 8.00 |
| | Sorbitan Sesquioleate | 1.00 |
| | Lanolin | 2.00 |
| | BHT | 0.02 |
| | | |
| 2 | (all-E)-3,3'-astaxanthin-dimethyldisuccinate | 0.05 |
| | Pigment | 5.00 |
| | | |
| | 3 Fragrance | 0.40 |

Procedure: Heat part 1 to 85°C while stirring, until complete dissolution- Let the mixture cool down to 60°C then add part 2 while mixing. Cool to ambient temperature. Then pass the preparation three times through a three roller mill. Heat again to 70°C, add part 3 while mixing. Pour the hot preparation into jars.

### Example 11 : Liptone with sunscreens

| Phase | *INCl Nomenclature* | % *w* / *w* |
|---|---|---|
| 1 | Ethylhexyl Methoxycinnamate | 6.00 |
| | Butyl Methoxydibenzoylmethane | 2.00 |
| | 4-Methylbenzylidene Camphor | 3.00 |
| | lsostearyl Neopentanoate | 5.00 |
| | Cyclomethicone | 5.00 |
| | Castor Oil | Ad 100 |
| | Coco-CaprylatelCaprate | 15.00 |
| | Euphorbia Cerifera (Candelilla) Wax | 5.00 |
| | Beeswax | 7.50 |
| | Dimethicone Copolyol | 4.00 |
| | Polydecene | 8.00 |
| | Copemicia Cerifera (Camauba) Wax | 5.00 |
| | Sorbitan Sesquioleate | 1.00 |
| | Lanolin | 2.00 |
| | BHT | 0.02 |
| | | |
| 2 | (all-E)-3,3'-astaxanthin-dimethyldisuccinate | 0.10 |
| | Pigment | 5.00 |
| | | |
| 3 | Fragrance | 0.40 |

Procedure: Heat part 1 to 85°C while stirring, until complete dissolution. Let the mixture cool down to 60°C then add part 2 while mixing. Cool to ambient temperature. Then pass the preparation three times through a three roller mill. Heat again to 70°C, add part 3 while mixing. Pour the hot preparation into jars.

### Example 12: Hard gelatin capsule

Hard gelatin capsules are prepared by conventional procedures providing a dose of (all-E)-3,3'-astaxanthin-dimethyldisuccinate of 20 mg. A suitable daily dose is 1 to 5 capsules.
Other ingredients:
Fillers: lactose or cellulose or cellulose derivatives q.s.
Lubricant: magnesium stearate if necessary (0.5%)

## Claims

1. Use of an astaxanthin derivative represented by general formula (I) 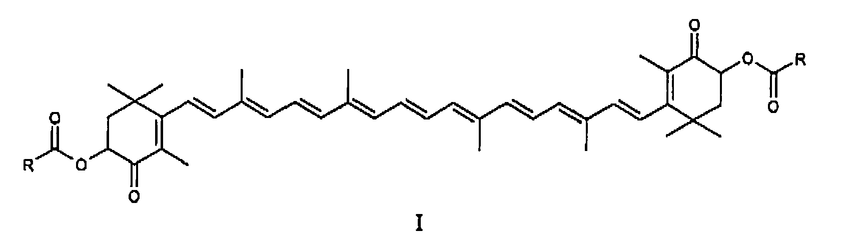 wherein
R is in each case a group (a), (b) or (c)
-NH-CH(R¹)-COOR² (a)
-OR³ (b)
-(Y)ₙ-Z (c)
R¹ signifies hydrogen or the residue of a protein-forming amino acid,
R² signifies C₁₋₆-alkyl or C₃₋₈-cycloalkyl,
R³ signifies C₁₋₁₂-alkyl or C₃₋₈-cycloalkyl,
n signifies zero or 1,
Y signifies C₁₋₇-alkylene or C₂₋₇-alkenylene,
and Z ,when n is zero, signifies C₃₋₈-cycloalkyl, a group -CH(C₆H₅)OR⁴, a group -COR⁵ or a group -CH₂N⁺(CH₃)₃ Hal⁻,
or Z ,when n is 1, signifies amino, a group -O-COR⁶, a group -NHCOR⁶, a group -OR⁷ or a group -SR⁸,
or Z regardless of whether n is zero or 1, signifies alternatively aryl, heteroaryl, a group, -COOR⁵ or a group -CH(CH₃)OR⁴,
R⁴ signifies hydrogen or acetyl,
R⁵ signifies hydrogen or C₁₋₆-alkyl,
R⁶ signifies C₁₋₆-alkyl, aryl or heteroaryl,
R⁷ signifies hydrogen, C₁₋₆-alkyl or acetyl,
R⁸ signifies C₁₋₆-alkyl, and
Hal⁻ signifies a halogen ion
for providing a cosmetic effect.

2. The use of an astaxanthin derivative according to claim 1, wherein the astaxanthin derivative is selected from
astaxanthin-diethyldicarbonate (R is ethoxy),
astaxanthin-diethyldioxalate (R is ethoxycarbonyl),
astaxanthin-di(N-acetylglycinate) (R is acetyfaminomethyl),
(all-E)-3,3'-rac-astaxanthin-dimaleinate (R is -CH=CH-COOH),
(all-E)-3,3'-rac-astaxanthin-disuccinate (R is -CH₂-CH₂-COOH),
(all-E)-3,3'-astaxanthin-dimethyldisuccinate (R is -CH₂-CH₂-COOCH₃),
(all-E)-3,3'-rac-astaxanthin-diethyldiglycinedicarbamate (R is -NH-CH₂-COOC₂H₅),
(all-E)-3,3'-rac-astaxanthin-dinicotinate (R is 3-pyridyl), (all-E)-3,3'-rac-astaxanthin-dimethioninedicarbamate (R is -NHCH(CH₂CH₂SCH₃)COOC₂H₅),
(all-E)-3,3'-rac-astaxanthin-diacetyldiglycolate (R is acetyloxymethyl),
(all-E)-3,3'-rac-astaxanthin-diphenylalaninedicarbamate (R is -NHCH(CH₂C₆H₅)COOC₂H₅),
(all-E)-3,3'-rac-astaxanthin-diethyfdifumarate (R is -CH=CH-COOC₂H₅),
(all-E)-3,3'-rac-astaxanthin-difuran-2-carbonate (R is 2-furyl),
(all-E)-3,3'-rac-astaxanthin-dimethyldimalonate (R is -CH₂-COOCH₃),
(all-E)-3,3'-rac-astaxanthin-di(3-methylthiopropionate) (R is 3-methylthioethyl),
(all-E)-3,3'-rac-astaxanthin-dimethoxyacetate (R is methoxymethyl),
(all-E)-3,3'-rac-astaxanthin-di-[(2-thienyl)acetate] [R is (2-thienyl)methyl],
astaxanthin-dilactate (R is 1-hydroxyethyl),
astaxanthin-di(acetylmandelate) (R is α-acetyloxybenzyl),
(all-E)-3,3'-rac-astaxanthin-dihippurate (R is benzoylaminomethyl) and
(all-E)-3,3'-rac-astaxanthin dibetainate [R is -CH₂N⁺(CH₃)₃ Cl⁻].

3. The use according to claim 1 or 2, wherein the astaxanthin derivative is (all-E)-3,3'-astaxanthin-dimethyldisuccinate (R is -CH₂-CH₂-COOCH₃).

4. The use according to any of the preceding claims, wherein the compound of formula (I) is used for beautification and improvement of mammalian skin, hair and nails.

5. The use according to any of claims 1 to 4, wherein the astaxanthin derivative of formula (I) is used for prevention and/or treatment of wrinkles, exfoliating skin, for thickening of the epidermis, as a moisturizer and/ or for maintaining a healthy skin flora and a proper skin functionality.

6. The use according to any of claims 1 to 5, wherein the astaxanthin derivative of formula (I) is used for prevention and/or treatment of wrinkles.

7. A cosmetic composition comprising an astaxanthin derivative of formula (I) as defined in any of claims 1 to 3 and a vehicle or carrier conventionally used in cosmetic compositions.

8. The composition according to claim 7, wherein the composition is an oral or topical preparation, preferably a topical composition.

9. The composition according to claim 7, wherein the composition is a decorative preparation.

10. The composition according to claim 7 wherein at least one additional active agent that is different from the astaxanthin derivative of formula (I) is present which is selected from anti-wrinkle/anti-atrophy/anti-ageing actives, other alpha hydroxyl acids, anfi-oxidants/radical-scavengers, flavonoids, anti-cellulite agents, tanning actives, skin-lightening agents, sunscreen actives, particulate matter, hair growth actives, penetration enhancers/delivery agents, skin soothing actives, anti-acne actives, fragrances, dyes, preservatives, emulsifiers, co-emuisifieres and pigments.

11. The composition according to any of claims 7 to 10, wherein the astaxanthin derivative of formula (I) is contained in the composition in a concentration of 0.001 to 20 wt.-%, based on the total weight of the composition.
